# EUROPEAN PATENT APPLICATION

(11) **EP 3 372 215 A1**
(43) Date of publication of application: **12.09.2018**
(21) Application number: 17187526.3
(22) Date of filing: 23.08.2017
(51) Int. Cl.: A61G 7/057

(54) **IMPROVED PRESSURE RELIEVING MATTRESS**

(30) Priority: 10.03.2017 GB 201703882
(71) Applicant: Select Medical Limited, Whitebirk Industrial Estate Blackburn Lancashire BB1 5NA (GB)
(72) Inventor: BOOTH, Ray, Blackburn, Lancashire BB2 7EX (GB); BARTON, Craig, Chorley, Lancashire PR6 7HT (GB)
(74) Representative: HGF Limited

(57) **Abstract**

A pressure relieving support having a top side configured to be adjacent a user and a bottom side opposite the top side, and comprising at least one selectively inflatable cell. The cell has a first inflatable portion positioned adjacent the bottom side and a second inflatable portion positioned adjacent the top side. One of the first inflatable portion and the second inflatable portion substantially surrounds a solid foam structure, and the other of the first inflatable portion and the second inflatable portion does not include a solid foam structure.

## Description

This invention relates to an improved pressure relieving support, and in particular to a pressure relieving support that may reduce the likelihood of pressure sores developing in patients supported on the pressure relieving support.

### BACKGROUND

If a patient is bedbound for a prolonged period of time then there is a risk that they may develop pressure sores (also known as pressure ulcers). Pressure sores are wounds that are caused by a continuous occlusion of blood vessels that supply oxygen and glucose to the tissues due to excessive pressure and/or shear. Pressure relieving mattresses may be used to reduce the likelihood of pressure sores developing in patients.

A pressure relieving mattress may be "static" or "active" (also referred to as dynamic or alternating). Static mattresses are typically made out of foam and work to spread a patient's weight over a large area. Active pressure relieving mattresses are powered devices that periodically redistribute pressure by repeatedly loading and unloading the pressure beneath the patient's body. This may be achieved by cyclically inflating and deflating alternate air cells in the mattress.

It is also known to provide a so-called "hybrid" mattress that includes an array of inflatable air cells where each air cell includes a foam core. Whilst the intention of such hybrid mattresses is to provide benefits traditionally associated with both static and active mattresses, many known hybrid mattresses fail to provide such benefits to a satisfactory degree.

It is an object of certain embodiments of the present invention to provide an improved pressure relieving support that may overcome certain disadvantages associated with the prior art.

### BRIEF SUMMARY OF THE DISCLOSURE

In accordance with a first aspect of the present invention there is provided a pressure relieving support having a top side configured to be adjacent a user and a bottom side opposite the top side, comprising:
at least one selectively inflatable cell;
wherein the cell has a first inflatable portion positioned adjacent the bottom side and a second inflatable portion positioned adjacent the top side, and
wherein one of the first inflatable portion and the second inflatable portion substantially surrounds a solid foam structure, and the other of the first inflatable portion and the second inflatable portion does not include a solid foam structure.

The first inflatable portion may underlie the second inflatable portion.

The second inflatable portion may include the solid foam structure, and the first inflatable portion does not include a solid foam structure. The first inflatable portion may be fluidly connected to the second inflatable portion. Alternatively, the first inflatable portion may be fluidly isolated from the second inflatable portion, so that the second inflatable portion encloses the solid foam structure.

The at least one selectively inflatable cell may include a wall that at least partially separates the first inflatable portion and the second inflatable portion.

In certain embodiments, the at least one cell is elongate.

When the cell is inflated, a cross-section of the cell may have a profile including a waist section between the first inflatable portion and the second inflatable portion, the waist being narrower in width than adjacent sections of the first inflatable portion and the second inflatable portion respectively. In the cross-section of the cell, the wall may be positioned across the waist section of the cell.

In certain embodiments, the solid foam structure may be elongate. A cross-section of the solid foam structure may have a quadrilateral profile.

The solid foam structure may have a length less than or substantially equal to a length of the cell surrounding the solid foam structure.

The pressure relieving support may further comprise a cover configured to at least partially enclose the at least one selectively inflatable cell. The cover may comprise a plastics material, which may be a polyurethane material.

The at least one selectively inflatable cell may comprise an array of said selectively inflatable cells. Each cell of the array of selectively inflatable cells may be adjacent lengthways to at least one other cell of the array. At least a first section of the array of selectively inflatable cells may be arranged so that a length of each of the cells is parallel to a length of the pressure relieving support. The pressure relieving support may further comprise a second section of the array of selectively inflatable cells, the second section of the array being arranged so that a length of each of the cells of the second section is parallel to a width of the pressure relieving support. The second section of the array may be positioned between the first section of the array and an end of the pressure relieving support.

The pressure relieving support may further comprise a base adjacent the bottom side, the base having a length, a width and a height. The at least one selectively inflatable cell may be attached to the base. The at least one selectively inflatable cell may be positioned above and adjacent to the base such that the base at least partially determines the profile of the at least one selectively inflatable cell. Along the width of the base from a first side of the base towards a second side of the base that is opposite the first side, the height of the base may generally increase and then generally decreases. In certain embodiments, the base may have a bottom surface that is substantially flat. The base may have a substantially uniform cross-section throughout its length.

The pressure relieving support may further comprise a first sidewall extending along the length of the pressure relieving support, and a second sidewall extending along the length of the pressure relieving support opposite the first sidewall, wherein the base, the first sidewall and the second sidewall are configured to delimit the at least one selectively inflatable cell. The first sidewall and/or the second sidewall may be contiguous with the base.

In accordance with a second aspect of the present invention there is provided a pressure relieving support having a top side configured to be adjacent a user and a bottom side opposite the top side, comprising:
a base adjacent the bottom side, the base having a length, a width and a height; and
an array of selectively inflatable cells positioned above and adjacent to the base towards the top side such that the base at least partially determines the profile of the array of cells;
wherein, along the width of the base, from a first side the height of the base generally increases and then generally decreases towards a second side opposite the first side.

The base may have a bottom surface that is substantially flat. The base may have a substantially uniform cross-section throughout its length.

The array of selectively inflatable cells may comprise at least one selectively inflatable cell having a first inflatable portion positioned adjacent the bottom side and a second inflatable portion positioned adjacent the top side. One of the first inflatable portion and the second inflatable portion may substantially surround a solid foam structure. In certain embodiments, the other of the first inflatable portion and the second inflatable portion may not include a solid foam structure. In particular embodiments, the second inflatable portion may include the solid foam structure, and the first inflatable portion does not include a solid foam structure. The first inflatable portion may be fluidly connected to the second inflatable portion. Alternatively, the first inflatable portion is fluidly isolated from the second inflatable portion. The second inflatable portion may enclose the solid foam structure.

Each cell of the array may include a wall that at least partially separates the first inflatable portion and the second inflatable portion. The first inflatable portion may underlie the second inflatable portion. Each cell of the array may be elongate.

When the cell is inflated, a cross-section of the cell may have a profile including a waist section between the first inflatable portion and the second inflatable portion, the waist being narrower in width than adjacent sections of the first inflatable portion and the second inflatable portion respectively. In the cross-section of the cell, the wall may be positioned across the waist section of the cell.

The solid foam structure may be elongate.

A cross-section of the solid foam structure may have a quadrilateral profile.

The solid foam structure may have a length less than or substantially equal to a length of the at least one selectively inflatable cell.

The pressure relieving support may further comprise a cover configured to at least partially enclose the array. The cover may comprise a plastics material which may be a polyurethane material.

Each cell of the array may be substantially elongate and adjacent lengthways to at least one other cell of the array. At least a first section of the array of selectively inflatable cells may be arranged so that a length of each of the cells is parallel to a length of the pressure relieving support. The pressure relieving support may further comprise a second section of the array of selectively inflatable cells, the second section of the array being arranged so that a length of each of the cells of the second section is parallel to a width of the pressure relieving support.

The second section of the array may be positioned between the first section of the array and an end of the pressure relieving support.

The pressure relieving support may further comprise a first sidewall extending along the length of the pressure relieving support, and a second sidewall extending along the length of the pressure relieving support opposite the first sidewall, wherein the base, the first sidewall and the second sidewall are configured to delimit the array. The first sidewall and/or the second sidewall may be contiguous with the base.

The array of selectively inflatable cells may be attached to the base.

In accordance with a third aspect of the present invention, there is provided a pressure relieving support having a length and a width, comprising:
an array of selectively inflatable cells; and
a solid foam structure enclosed by at least one of the cells of the array;
wherein each cell of the array is substantially elongate and adjacent lengthways to at least one other cell of the array; and
at least a first section of the array is arranged so that a length of each of the cells of the array is parallel to the length of the pressure relieving support.

The pressure relieving support may further comprise a second section of the array of selectively inflatable cells arranged so that a length of each of the cells of the second section is parallel to the width of the pressure relieving support. The second section of the array may be positioned between the first section of the array and an end of the pressure relieving support.

The pressure relieving support may further comprise a base, the base having a length, a width and a height. The array of selectively inflatable cells may be attached to the base.

The pressure relieving support may further comprise a first sidewall extending along the length of the pressure relieving support, and a second sidewall extending along the length of the pressure relieving support opposite the first sidewall, wherein the base, the first sidewall and the second sidewall are configured to delimit the array of selectively inflatable cells.

The pressure relieving support may have a top side configured to be adjacent a user and a bottom side opposite the top side in use, wherein the array of selectively inflatable cells comprises at least one selectively inflatable cell having a first inflatable portion positioned adjacent the bottom side and a second inflatable portion positioned adjacent the top side.

The first inflatable portion may underlie the second inflatable portion. One of the first inflatable portion and the second inflatable portion may substantially surround a solid foam structure. The other of the first inflatable portion and the second inflatable portion may not include a solid foam structure. In certain embodiments, the second inflatable portion may include the solid foam structure, and the first inflatable portion does not include a solid foam structure. The first inflatable portion may be fluidly connected to the second inflatable portion. Alternatively, the first inflatable portion may be fluidly isolated from the second inflatable portion.

The second inflatable portion may enclose the solid foam structure.

The at least one selectively inflatable cell may include a wall that at least partially separates the first inflatable portion and the second inflatable portion.

When the cell is inflated, a cross-section of the cell may have a profile including a waist section between the first inflatable portion and the second inflatable portion, the waist being narrower in width than adjacent sections of the first inflatable portion and the second inflatable portion respectively. In the cross-section of the cell, the wall may be positioned across the waist section of the cell.

The solid foam structure may be elongate.

A cross-section of the solid foam structure may have a quadrilateral profile. The solid foam structure may have a length less than or substantially equal to a length of the cell.

The pressure relieving support may further comprise a cover configured to at least partially enclose the array of selectively inflatable cells. The cover may comprise a plastics material which may be a polyurethane material.

The pressure relieving support may have a top side configured to be adjacent a user and a bottom side opposite the top side, wherein the array of selectively inflatable cells may be positioned above and adjacent to the base towards the top side such that the base at least partially determines the profile of the array of cells. Along the width of the base from a first side of the base towards a second side of the base that is opposite the first side, the height of the base may generally increases and then generally decreases.

The base may have a bottom surface that is substantially flat. The base may have a substantially uniform cross-section throughout its length.

In any aspect of the invention, the pressure relieving support may be a pressure relieving mattress.

In any aspect of the invention, the pressure relieving support may further comprise a pump configured to inflate the inflatable cells.

In any aspect of the invention, the pressure relieving support may further comprise a controller for controlling the inflation of the inflatable cells.

### BRIEF DESCRIPTION OF THE DRAWINGS

Embodiments of the invention are further described hereinafter with reference to the accompanying drawings, in which:
Figure 1 is a schematic view of a pressure relieving mattress according to an embodiment of the present invention;
Figure 2 is a schematic view of a cross-section of a selectively inflatable cell according to an aspect of the present invention, wherein the cell is in an inflated configuration; and
Figure 3 is a cross-sectional view of a pressure relieving support including a base according to an aspect of the present invention.

### DETAILED DESCRIPTION

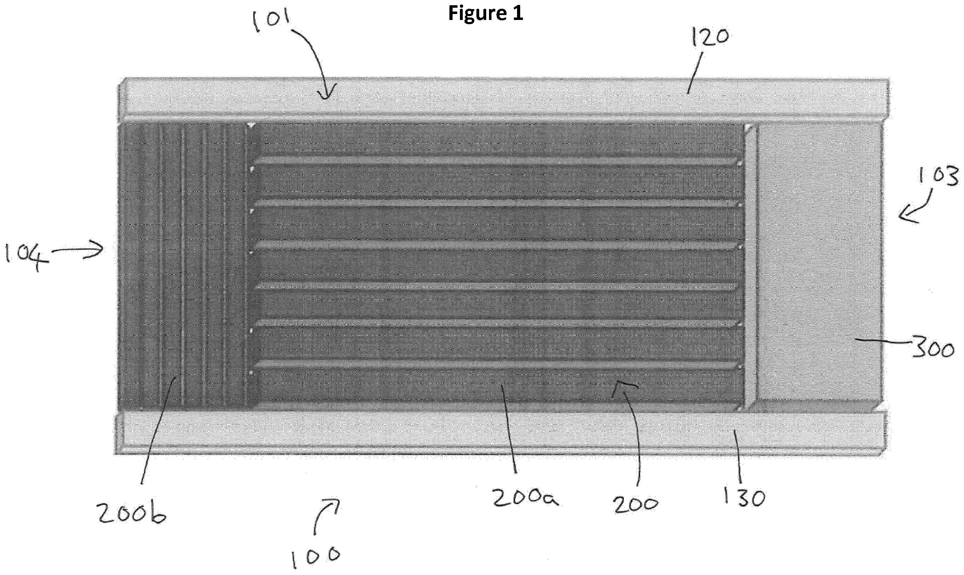

Figure 1 shows a pressure relieving support 100 according to an embodiment of the present invention. The pressure relieving support 100 described below with reference to the Figures is a mattress. However, in alternative embodiments the pressure relieving support may be any alternative support for supporting a person.

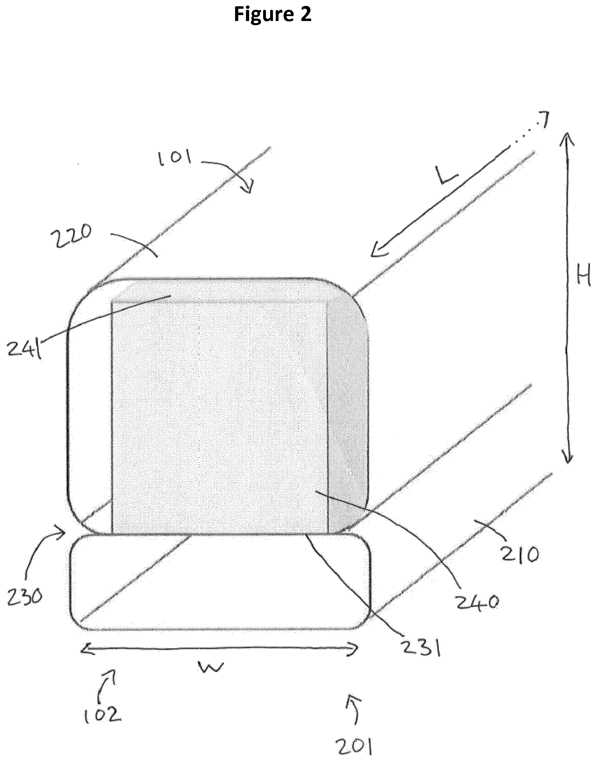

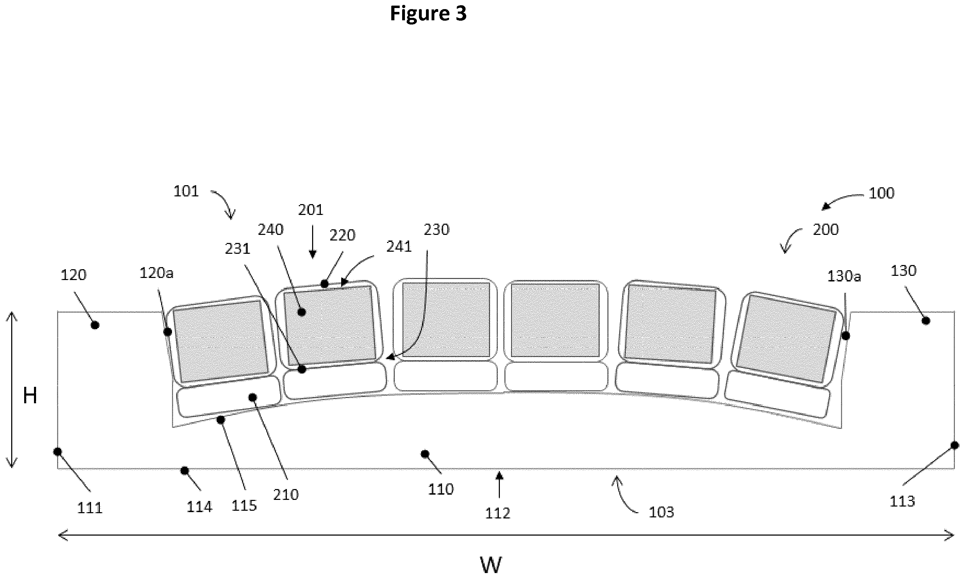

The pressure relieving support 100 has a base 110 (as seen in Figure 3), a first sidewall 120, a second sidewall 130 opposite to the first sidewall 120, an array of selectively inflatable cells 200, and a headrest 300. The base 110, the first sidewall 120 and the second sidewall 130 are configured to cooperatively delimit the array of selectively inflatable cells 200. The pressure relieving support 100 has a top side 101 and a bottom side 102 (as seen in Figures 2 and 3) opposite the top side 101. The top side 101 is configured to be adjacent the patient in use and provide a support surface, and the bottom side 102 is configured to rest on a surface, e.g. a bed or a floor. The pressure relieving support has a first end 103 and a second end 104 opposite the first end 103. The first end 103 is configured to be nearest to a patient's head, and the second end 104 is configured to be nearest a patient's feet.

The pressure relieving support 100 may comprise a cover (not shown) configured to at least partially enclose the array 200 so to provide a layer between the array 200 and a patient when the patient lies on the pressure relieving support 100. The cover may be removable and/or washable. The cover may comprise a plastics material, for example polyurethane, or any material suitable for use in providing a layer between the array 200 and the patient.

Figure 2 shows a schematic view of a cross-section of a selectively inflatable cell 201 according to an aspect of the present invention. The cell 201 is selectively inflatable by any means suitable for providing a fluidic pressure to inflate the cell 201, e.g. a pump. In Figure 2, the cell 201 is shown in an inflated configuration. The cell 201 may be inflated using any suitable fluid (which may be a liquid or a gas, e.g. air). Two or more selectively inflatable cells 201 may combine to form the array of selectively inflatable cells 200. In use, alternate cells 201 of the array 200 may be cyclically inflated and deflated simultaneously, so that adjacent cells 201 are either deflated or inflated, and the patient is always supported by at least part of the array 200.

The cell 201 comprises a first inflatable portion 210 and a second inflatable portion 220. The first inflatable portion 210 is configured to be adjacent the bottom side 102 of the pressure relieving support 100 and underlie at least a portion of the second inflatable portion 220. The second inflatable portion 220 is configured to be adjacent the top side 101 of the pressure relieving support 100 and overlie at least a portion of the first inflatable portion. As such, in use the patient would be adjacent to the second inflatable portion 220 of the cell 201 and be supported on the top side 101 of the pressure relieving support 100.

The first inflatable portion 210 is fluidly connected to the second inflatable portion 220 so that, in use, only one of the two portions 210, 220 needs to be fluidly connected to a fluid pressure source (not shown) to inflate the cell 201. This is advantageous in that the likelihood of one portion inflating out of sync with the other portion is substantially removed. Therefore, such an arrangement may improve the efficacy of the pressure relieving support 100.

The cell 201 is elongate in shape, as can be seen clearly in Figure 1. Thus the cell 201 has a length L, a width W and a height H, wherein the length L, the width W and the height H are perpendicular to one another and the length L is greater than each of the width W and the height H. In the array 200, each inflatable cell 201 is positioned adjacent lengthways to at least one other inflatable cell 201. In other embodiments, some cells 201 may be positioned end to end relative to one another.

In the cross-sectional view shown in Figure 2, it can be seen that the cross-sectional profile of the cell 201 has a shape that may be described as a figure-of-eight shape. That is to say, the outer shape of the cell 201 is figure-of-eight-shaped. The cell 201 comprises a wall 231 which separates the first inflatable portion 210 and the second inflatable portion 220 along a portion of the length of the cell 201. The wall 231 extends across a waist section 230 of the cell 201, as shown in Figure 2. The waist section 230 forms the narrow central portion of the figure-of-eight-shaped profile of the cell 201. That is to say, the waist section 230 is narrower in width than adjacent sections of the first inflatable portion 210 and the second inflatable portion 220 respectively, as can be seen clearly in Figure 2. As such, the first inflatable portion 210 forms a bottom part of the figure-of-eight-shaped profile, and the second inflatable portion 220 forms a top part of the figure-of-eight-shaped profile. The cross-sectional profile shown in Figure 2 extends substantially throughout the length L of the cell 201, i.e. the cross-section is substantially uniform.

In the cross-sectional view shown in Figure 2, each of the first inflatable portion 210 and the second inflatable portion 220 is generally quadrilateral-shaped and comprises rounded corners. In other embodiments, the first inflatable portion and/or the second inflatable portion may comprise any suitable cross-sectional shape so that the cell is generally figure-of-eight-shaped, wherein the waist section is narrower than the adjacent sections of the first inflatable portion and the second inflatable portion respectively.

One or more of the cells 201 may be secured to the base 110 of the pressure relieving support 100. The cross-sectional profile of the cell 201 described above may advantageously reduce the likelihood of the cell 201 unwantedly deforming in response to a sideways force on the second inflatable portion 220 of the cell 201. If the waist section was not present, and sides of the first inflatable portion were contiguous and parallel with sides of the second inflatable portion, then such a sideways force may cause the second inflatable portion to rotate relative to the first inflatable portion and collapse inwardly. Whilst embodiments having a waist section may be preferable, other embodiments of the invention may not include a waist section but still provide other advantages over the prior art.

In the embodiment shown in Figure 2, the wall 230 does not extend throughout the length L of the cell 201. As such, there exists one or more sections (not shown) configured to provide the fluid connection between the first inflatable portion 210 and the second inflatable portion 220. As such, in at least one cross-section of the cell 201 along its length L, the wall 231 is not present. In certain embodiments, the wall 231 is not present at each end of the elongate cell 201 to permit fluid flow between the first inflatable portion 210 and the second inflatable portion 220. In certain embodiments, the first inflatable portion 210 may be separated (at least in part) from the second inflatable portion 220 by one or more walls.

In an alternative embodiment of the invention, the first inflatable portion may not be fluidly connected to the second inflatable portion, i.e. the first inflatable portion and the second inflatable portion may be separate sub-cells of a larger cell. In such an embodiment, the wall may be present or a boundary may be determined between the first inflatable portion and the second inflatable portion by adjoining surfaces of the first inflatable portion and the second inflatable portion. The first inflatable portion and the second inflatable portion may each be inflated using independent or the same pumping means, and/or they may be inflated simultaneously or otherwise.

As shown in Figure 2, the second inflatable portion 220 comprises a solid foam structure 240 configured to extend substantially throughout the length L of the cell 201. The solid foam structure forms a foam "core" within the cell 201. Throughout the present specification, the term "solid foam structure" may refer to an open-cell foam or a closed-cell foam. An open-cell foam is one which a fluid may flow through the entire structure. A closed-cell foam is one which includes integral sealed cavities. The "solid foam structure" may include any suitable foam material, including but not limited to ether, high resilience, and visco-elastic foam.

The solid foam structure 240 is configured to relieve pressure on parts of the patient's body by distributing the weight of the patient when the patient is positioned on the cell 201, and, in particular when the cell 201 is in a deflated configuration.

In the cross-sectional view shown in Figure 2, the second inflatable portion 220 and the wall 230 collectively enclose the solid foam structure 220. In use, the solid foam structure 240 may rest on the wall 231, as shown in Figure 2. The cross-section of the solid foam structure 240 has a substantially quadrilateral profile so that a top surface 241 of the solid foam structure 240 is substantially flat. The cross-section of the solid foam structure 240 shown in Figure 2 extends throughout the length of the solid foam structure 240, i.e. the cross-section is uniform. Having a substantially flat top surface advantageously maximises the area in which parts of the patient's body may be supported, consequently improving the pressure relieving capability of the solid foam structure 240. In other embodiments, the solid foam structure may have any suitable cross-sectional profile suitable that relieves pressure on parts of the patient's body by distributing the weight of the patient. For example, the solid foam structure may have curved sides or a curved top surface.

In the above-described embodiment in which the second inflatable portion 220 is fluidly isolated from the first inflatable portion 210 (e.g. not fluidly connected, or selectively fluidly connected), the second inflatable portion 220 encloses the solid foam structure 240 throughout the length L of the cell 201.

The first inflatable portion 210 of the cell 201 does not include a solid foam structure. As such, the first inflatable portion 210 is empty (other than any fluid used to inflate the first inflatable portion 210), as shown clearly in Figure 2. Consequently, a large difference in vertical displacement may be achieved between the inflated and deflated configurations of the cell 201. Indeed, in the deflated configuration, the first inflatable portion 210 may collapse to have substantially zero volume and the second inflatable portion 220 may be vertically displaced downwardly by an amount up to the vertical height of the previously inflated first inflatable portion 210. The second inflatable portion 220 may not collapse to have a volume less than that permitted by the presence of the solid foam structure 240, and, as such, the solid foam structure 240 may provide support to a patient when the cell is in the deflated configuration.

In alternative embodiments, the first inflatable portion 210 may include the solid foam structure 240 and the second inflatable portion 220 may not include a solid foam structure to achieve a large difference in vertical displacement between the inflated and deflated configurations. However, it may be preferable for the second inflatable portion 220 to include the solid foam structure 240 since, otherwise, a deflated second inflatable portion 220 without a foam structure 240 may form a creased structure when deflated, and such creases may cause discomfort to a patient supported on the pressure relieving support 100 and/or increase the risk of pressure sores developing.

Some or all of the cells 201 may be attached to one or more of the base 110, the first sidewall 120, the second sidewall 130 and another cell 201. The cells 201 may be attached using temporary or permanent means e.g. Velcro ®, releasable clips or an adhesive.

In certain embodiments, the array 200 may feature cells 201 according to any of the above-described embodiments. For example, the array 200 may include one or more cells that have the solid foam structure in the second inflatable portion, and/or one or more cells that have the solid foam structure in the first inflatable portion, and/or one or more cells that have a fluidly connected first inflatable portion and second inflatable portion, and/or one or more cells that have a fluidly separate first inflatable portion and second inflatable portion.

Figure 3 shows a schematic cross-sectional view of the pressure relieving support 100 of Figure 1. According to an aspect of the invention, the base 110 has a length L, a width W and a height H, where the length L, width W and height H are all mutually perpendicular to one another. In a direction along the width W of the base 110, the height H of the base 110 has a convex profile 115. That is, in a direction along the width W of the base 110, from a first side 111 of the base 110 to a second side 113 of the base 110, at least a portion of the base 110 underlying the array 200 has a height H that generally increases towards a maximum (which may be a local maximum) and then generally decreases. In the embodiment shown in Figure 3, the maximum of the height is coincident with a longitudinal centre 112 of the base. In the embodiment shown in Figure 3, the convex profile 115 is curved. In other embodiments, the convex profile 115 may be formed by one or more straight lines. For example, the convex profile 115 may be generally triangular or stepped. In the schematic view shown in Figure 3, the dimensions of the convex profile 115 are greatly exaggerated to assist understanding of the invention.

The change in height of the convex profile 115 is preferably gradual so that the difference in height between the first side 111 or the second side 113 and the centre 112 is small. The convex profile 115 is configured to determine a generally convex profile of the array of selectively inflatable cells 200 overlying the base 110. As such, the array 200 may advantageously generally cause the user to rotate during each cycle of inflation and deflation, thus reducing the time in which the user is situated in one position. Consequently, the risk that the user may develop pressure induced ulcers is advantageously reduced. The convex profile 115 is preferably gradual so that the risk of the user rolling off the pressure relieving support 100 is substantially mitigated.

An inner surface 120a of the first sidewall 120 may be angled relative to a vertical axis to better accommodate and retain the array 200 due to profile of the array 200 determined by the convex profile 115. Similarly, an inner surface 130a of the second sidewall 130 may be angled relative to a vertical axis (but in an opposite direction to the inclination of the inner surface 120a of the first sidewall 120) for the same reasons.

Whilst in the preferred embodiment of this aspect of the invention, the array of selectively inflatable cells 200 comprises one or more of the cells 201 described above, certain aspects of the invention may relate to a pressure relieving support having a base with a convex profile and an array of any suitable inflatable cells overlying the convex profile of the base. In particular, an aspect of the present invention relates to a pressure relieving support having a top side configured to be adjacent a user and a bottom side opposite the top side, comprising:
a base adjacent the bottom side, the base having a length, a width and a height; and
an array of selectively inflatable cells positioned above and adjacent to the base towards the top side such that the base at least partially determines the profile of the array of cells;
wherein, along the width of the base, from a first side the height of the base generally increases and then generally decreases towards a second side opposite the first side.

In such aspects of the invention, the array of selectively inflatable cells 200 may comprise one or more cells wherein one or both of the first inflatable portion and the second inflatable portion comprise a solid foam structure, or where neither of the first inflatable portion nor the second inflatable portion comprises a solid foam structure. Indeed, in other embodiments the array 200 may comprise cells having a single inflatable portion.

In the embodiment of Figure 3, the base 110 has a flat bottom surface 114 and thus the convex profile 115 determines the height of the base 110 along the width W of the cross-section. The base 110 has a uniform cross-section along the length L of the base 110 (which extends into the page when considering Figure 3). In other embodiments, the base 110 may not have a uniform cross-section along the length L, and instead the base may have a varying cross-section so as to determine a convex profile of at least a portion of the array 200. In other embodiments, the bottom surface 114 of the base 110 may be sloped so long as the base 110 determines a substantially convex profile of the array 200.

In other embodiments, the base 110 may comprise two or more convex profiles across the width W of the base 110, so long as the base 110 may determine the profile of the array 200 such that a patient may experience some movement (e.g. a rolling motion) during periodic inflation and deflation of the cells.

In certain embodiments, the headrest 300 and/or the sidewalls 120, 130 may not be provided.

According to another aspect of the invention, the array of cells 200 includes a plurality of cells 201 adjacent one another lengthways, and a first section 200a of the array 200 is arranged so that the length L of each cell 201 is parallel to a length L of the pressure relieving support 100. That is to say, the first section 200a of the array of cells 200 is arranged longitudinally, as shown in Figure 1. In particular, there may be provided a pressure relieving support having a top side configured to be adjacent a user and a bottom side opposite the top side, comprising:
a base adjacent the bottom side, the base having a length, a width and a height; and
an array of selectively inflatable cells positioned above and adjacent to the base towards the top side such that the base at least partially determines the profile of the array of cells;
wherein, along the width of the base, from a first side the height of the base generally increases and then generally decreases towards a second side opposite the first side.

Whilst in a preferred embodiment of this aspect of the invention, the array of selectively inflatable cells 200 (including the first section 200a) comprises one or more of the cells 201 described above, in alternative embodiments the array of selectively inflatable cells 200 may include any suitable pressure relieving cell where at least a portion of the cell contains a solid foam structure. For example, in embodiments of this aspect of the invention, the array of selectively inflatable cells 200 may comprise one or more cells wherein both the first inflatable portion and the second inflatable portion comprise a solid foam structure, so long as the length of the cells is parallel with the length L of the pressure relieving support 100.

The presence of longitudinally aligned cells 201 may advantageously provide better comfort for a patient supported on the pressure relieving support 100, and may advantageously improve the pressure relief provided by the pressure relieving support 100. The improved comfort and therapeutic effects of pressure relieving supports according to this aspect of the invention are further enhanced when the convex profile 115 described above in relation to Figure 3, and/or the cells 200 described above in relation to Figure 2 are also employed.

Significantly, the arrangement of longitudinally arranged air cells may prevent the patient from falling between the air cells, and provide a much more all-round comfortable support surface. This arrangement promotes a reduction of interface pressure across both 'static' and 'active' states and reduces friction and shear forces between adjacent cells, in use (i.e. when loaded), compared with horizontally arranged cells. Inflated air cell pressures in the 'active' (i.e. inflated) state can remain low and maintain comfort levels equivalent to those provided in the 'static' (i.e. deflated) state. This effect is particularly apparent when the longitudinal cells are arranged to support the torso of the patient.

In other embodiments the array 200 may further include a second section 200b wherein the length L of each cell 201 is parallel to a width W of the pressure relieving support 100. That is to say, the second section 200b of the array of cells 200 is arranged transversely, as shown in Figure 1. The second section 200b is positioned towards a second end 104 of the pressure relieving support 100, and the first section 200a is positioned between the second section 200b and the first end 103 of the pressure relieving support 100. The second section 200b may be configured to support the feet and/or lower limbs of a patient supported on the pressure relieving support 100. The second section 200b may advantageously have narrower cells 200 (i.e. in a direction transverse the length of the cells) for supporting the feet and/or lower limbs of a patient, relative to the cells of the first section 200a. Narrower cells may increase the number of cells that the feet and/or lower limbs of a patient are supported by. As such, a cyclic inflation and deflation of alternately arranged narrow cells may maintain adequate, but alternating support of the feet and/or lower limbs. In contrast, if the cells supporting the feet and/or lower limbs are too wide (e.g. such that a foot is supported on a single cell), little or no support will be provided to the feet and/or lower limbs when the cells are in a deflated configuration (e.g. if no support is provided by adjacent inflated cells).

In certain embodiments, the pressure relieving support 100 includes the headrest 300 positioned between the first section 200a and the first end 103. The headrest 300 may comprise a foam material, or any material suitable to support the head of the patient. In certain embodiments, the headrest 300 may be formed by an array of inflatable cells (which may or may not include a solid foam structure therein).

In other embodiments, the array may include more than one of either or both of the first section and the second section in any combination.

As noted above, in preferable embodiments the pressure relieving support is a pressure relieving mattress. In certain embodiments, the pressure relieving support may be provided with a pump or other air moving means to inflate one or more of the inflatable cells. In certain embodiments, the pressure relieving support may include a controller to control the inflation of the one or more inflatable cells. For example, the controller may control the operation of a pump or other air moving means.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A pressure relieving support having a top side configured to be adjacent a user and a bottom side opposite the top side, comprising:
at least one selectively inflatable cell;
wherein the cell has a first inflatable portion positioned adjacent the bottom side and a second inflatable portion positioned adjacent the top side, and
wherein one of the first inflatable portion and the second inflatable portion substantially surrounds a solid foam structure, and the other of the first inflatable portion and the second inflatable portion does not include a solid foam structure.

2. A pressure relieving support according to claim 1, wherein the first inflatable portion underlies the second inflatable portion.

3. A pressure relieving support according to claim 1 or 2, wherein the second inflatable portion includes the solid foam structure, and the first inflatable portion does not include a solid foam structure.

4. A pressure relieving support according to any preceding claim, wherein the first inflatable portion is fluidly connected to the second inflatable portion.

5. A pressure relieving support according to any preceding claim, wherein the at least one selectively inflatable cell includes a wall that at least partially separates the first inflatable portion and the second inflatable portion.

6. A pressure relieving support according to any preceding claim, wherein, when the cell is inflated, a cross-section of the cell has a profile including a waist section between the first inflatable portion and the second inflatable portion, the waist being narrower in width than adjacent sections of the first inflatable portion and the second inflatable portion respectively.

7. A pressure relieving support according to any preceding claim, wherein the solid foam structure has a length less than or substantially equal to a length of the cell surrounding the solid foam structure.

8. A pressure relieving support according to any preceding claim, further comprising a cover configured to at least partially enclose the at least one selectively inflatable cell.

9. A pressure relieving support according to any preceding claim, wherein the at least one selectively inflatable cell comprises an array of said selectively inflatable cells.

10. A pressure relieving support according to claim 9, wherein each cell of the array of selectively inflatable cells is adjacent lengthways to at least one other cell of the array; optionally
wherein at least a first section of the array of selectively inflatable cells is arranged so that a length of each of the cells is parallel to a length of the pressure relieving support; and optionally
wherein the pressure relieving support further comprises a second section of the array of selectively inflatable cells, the second section of the array being arranged so that a length of each of the cells of the second section is parallel to a width of the pressure relieving support.

11. A pressure relieving support according to any preceding claim, further comprising a base adjacent the bottom side, the base having a length, a width and a height.

12. A pressure relieving support according to claim 11, wherein the at least one selectively inflatable cell is attached to the base.

13. A pressure relieving support according to claim 11 or 12, wherein the at least one selectively inflatable cell is positioned above and adjacent to the base such that the base at least partially determines the profile of the at least one selectively inflatable cell.

14. A pressure relieving support according to any one of claims 11 to 13, wherein along the width of the base from a first side of the base towards a second side of the base that is opposite the first side, the height of the base generally increases and then generally decreases.

15. A pressure relieving support according to any one of claims 11 to 14, further comprising a first sidewall extending along the length of the pressure relieving support, and a second sidewall extending along the length of the pressure relieving support opposite the first sidewall, wherein the base, the first sidewall and the second sidewall are configured to delimit the at least one selectively inflatable cell.
